# EUROPEAN PATENT SPECIFICATION

(11) **EP 4 393 461 B1**
(45) Date of publication and mention of the grant of the patent: **19.03.2025**
(21) Application number: 23213826.3
(22) Date of filing: 01.12.2023
(51) Int. Cl.: A61F 13/26

(54) **APPLICATOR FOR FEMININE HYGIENE TAMPONS**
APPLIKATOR FÜR TAMPONS FÜR DIE FRAUENHYGIENE
APPLICATEUR POUR TAMPONS D'HYGIÈNE FÉMININE

(30) Priority: 30.12.2022 IT 202200027234
(43) Date of publication of application: 03.07.2024
(73) Proprietor: Corman S.p.A., 20084 Lacchiarella (IT)
(72) Inventor: Mantovani, Giorgio, Milano (IT); Vitale, Adriano, Milano (IT)
(74) Representative: Rastelli, Franco

(56) References cited:
- US-A1- 2003 105 421
- US-A1- 2007 276 317
- US-A1- 2016 185 955
- US-A1- 2021 079 211

## Description

### BACKGROUND OF THE INVENTION

The present invention relates to an applicator for feminine hygiene tampons.

As is known, plastic products are versatile and lightweight and can be produced at relatively low costs.

Currently, only around 1% of plastics and plastic products on the global market are considered to be bio-based, compostable and/or biodegradable.

Most plastics continue to be produced from fossil fuels in a process that contributes to increasing greenhouse gas emissions along their value chain.

In fact, plastic pollutes throughout its life cycle, from its production to use through to its disposal.

Plastic recycling rates are low and plastic enters the environment through, for example, littering, improper waste management and wear and tear on products which can remain in the environment for many years and may potentially enter the food chain.

In light of the above, it is clear that any non-biodegradable plastic product which is used continually and intensely can be a significant source of environmental pollution.

In the case of feminine hygiene tampon applicators, these are essentially made up of a syringe, wherein the cylinder containing the tampon and the plunger for expelling the tampon from the cylinder are currently made of plastic, and so they are a significant source of environmental pollution.

Publication US 2016/185955 A1 discloses compositions based on polylactic acid, polybutylene succinate and polybutyrate for the production of hygiene tampon applicators.

### SUMMARY OF THE INVENTION

The aim of the present invention is to eliminate the above-mentioned drawbacks relating to the prior art.

Within this aim, one object of the invention is to provide an applicator for feminine hygiene tampons made of a material that will not be a source of environmental pollution.

Another object of the invention is to provide an applicator for feminine hygiene tampons which, despite being made of a biodegradable and therefore nonpolluting material, offers the same functional characteristics as a conventional applicator made of plastic material.

A further object of the invention is to create an applicator for feminine hygiene tampons which is structurally the same as the applicators made of plastic material currently on the market.

These and other objects are achieved with the applicator of claim 1. Preferred embodiments of the invention will be apparent from the remaining claims.

### BRIEF DESCRIPTION OF THE DRAWINGS

The characteristics and advantages of the invention will be more evident from the following indicative and not exhaustive description of preferred but not exclusive embodiments of the applicator for feminine hygiene tampons illustrated in the following figures, in which:

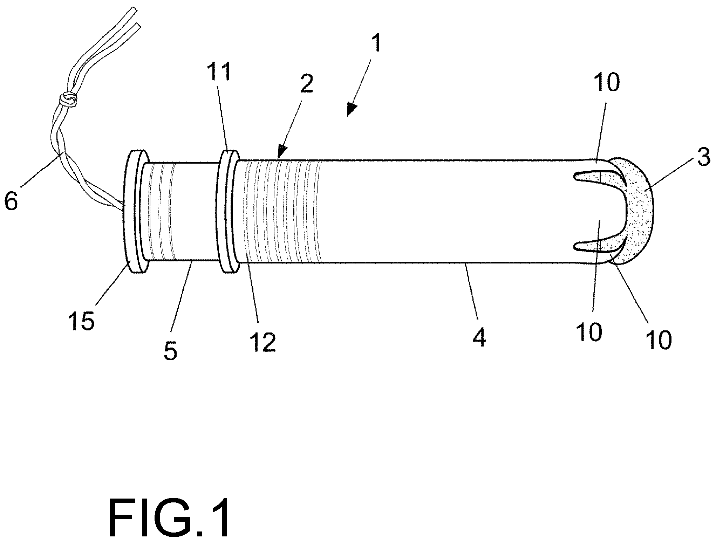

Figure 1 is a side elevation view of the applicator according to the invention in a closed condition;

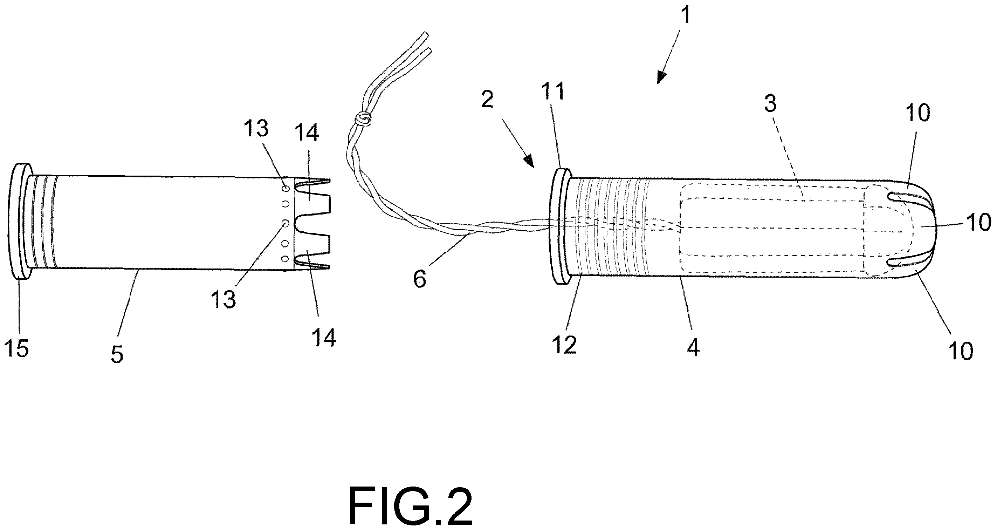

Figure 2 is an exploded, side elevation view of the applicator, with the tampon positioned inside the cylinder;
Figure 3 shows the curves of CO₂ produced (in grams) by the syringe of the applicator of the invention, in relation to the microcrystalline cellulose reference;
Figure 4 illustrates the aerobic biodegradability trend, under controlled compostability conditions, for the material making up the syringe 2 in relation to the microcrystalline cellulose sample.

### DETAILED DESCRIPTION OF THE PREFERRED EMBODIMENTS

With particular reference to the figures described above, the feminine hygiene tampon applicator of the invention is generically indicated with reference number 1.

In particular, the applicator 1 is of the type suitable for applying a feminine hygiene tampon 3 and includes a syringe 2 for expelling the tampon, for example made of very pure cotton wool, or of mixtures of rayon and cotton and the like. The tampon 3 has a hanging thread 6 to facilitate its removal after use.

The syringe 2 has a cylinder 4 containing the tampon 3 and a plunger 5 for expelling the tampon 3 from the cylinder upon its application.

Advantageously, the cylinder 4 or the plunger 5, or both, are made of a material that is both biodegradable and compostable.

Thanks to this technical solution, the syringe 2 is able to biodegrade in a specific medium (water, soil, compost) under certain conditions and in variable periods of time.

At the same time, the syringe is also compostable as it will biodegrade under the conditions of an industrial composting plant or an industrial anaerobic digestion plant with a subsequent composting stage.

This solution eliminates any environmental pollution.

In particular, the material used to make the plunger 5 and/or the cylinder 4 includes:
- polylactic acid with formula: (C3H6O3)ₙ
- polybutyrate with formula 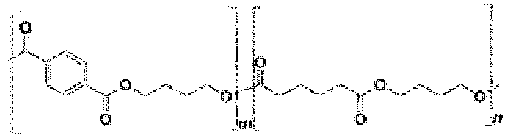
- polybutylene with formula (C8H12O4)ₙ

In a preferred embodiment of the invention, the material for making the aforementioned components of the syringe 2 also includes talc and additives such as epoxidized soybean oil and/or acetyl tributyl citrate and calcium stearate.

In greater detail, the material that offers the best biodegradability performance within the scope of the applicator of the invention and with valid structural resistance properties includes 3% to 10% by weight of polylactic acid, 34% to 60% by weight of polybutyrate, 10% to 15% by weight of polybutylene succinate, 20% to 30% by weight of talc and 7% to 11% by weight of epoxidized soybean oil and/or acetyl tributyl citrate and calcium stearate.

A preferred example of composition of the material of the invention is set out below:
Polylactic acid 7%
Polybutyrate 47%
Polybutylene succinate 13%
Talc 25%
Epoxidized soybean oil 3%
Acetyl tributyl citrate 3%
Calcium stearate 2%

The cylinder 4 of the applicator 1 of the invention has one end provided with flexible chamfers or tongues 10, suitable for allowing the tampon 3 to gradually exit from the syringe 2, in a unidirectional and frictional manner.

Said cylinder 4 has, on its opposite end, a grip collar 11 and inside it a circular groove 12 into which protuberances 13 present on the end of the plunger 5 snap engage.

On the same end there are also flexible fingers 14 which serve to push the tampon 3 uniformly out of the cylinder 4 of the applicator.

On the opposite end the plunger 5 has a raised collar 15 which facilitates its movement by the user.

To determine the aerobic biodegradability of the syringe 2 of the applicator of the invention, controlled compostability conditions in accordance with the UNI EN ISO 14855-1:2013 standards were applied.

The determination of Total Organic Carbon (TOC) was carried out in accordance with UNI EN 13137:2002 "Determination of total organic carbon (TOC) in waste, sludges and sediments".

Microcrystalline cellulose in powder form with a TOC equal to 42.2% was used as a reference material.

### MATERIALS

### Characteristics of the sample of the invention

- Name: "Compact feminine tampon applicators EarthBi AB313_LT"
- Description: applicator for feminine hygiene tampons
- Color: white
- Dry weight: 99.5%
- Volatile solids: 89.7%
- Total organic carbon (TOC): 51.0%
Sample ground at the start of the test

### Characteristics of the reference sample

- Name: Cellulose
- Product: microcrystalline cellulose
- Physical form: powder
- Color: white
- Batch number: MKCG8490
- Brand: Aldrich
- Total organic carbon (TOC): 42.2%

### Biodegradability test general procedure

In accordance with the UNI EN ISO 14855-1:2013 standard, the test is carried out by mixing the sample with mature compost in order to verify whether the microbial environment of the compost is capable of breaking down the organic fraction of the sample into CO₂, water and biomass.

The inoculum consists of mature compost obtained from an industrial composting plant. The compost is sieved to eliminate the coarse fraction. The fine fraction obtained represents the inoculum for the test, whose volatile solids content must be greater than 30% of the value of the total volatile solids.

The sample under analysis (or the reference sample) is mixed with the inoculum in a ratio of 1:6 (dry weight) and introduced into the reactor. The reactors are placed in an incubator at a temperature of 58 ± 2°C for the entire duration of the test. The aerobic conditions are maintained by blowing ambient air into the reactors. Aerobic conditions allow the conversion of the organic fraction of the sample into CO₂ during the test. The air flow at the outlet of each reactor is sent to a gas analyzer which determines the concentration of CO₂ and the flow at the reactor outlets at regular time intervals. The rate of biodegradation is determined as the percentage of the theoretical initial organic carbon content of the sample that is converted into CO₂.

### Test conditions

### Test setup

### Sample preparation:

The sample was cut and ground until a thin, homogeneous fluff was obtained.

### Reactor setup:

Blank: three glass reactors (3 liter capacity) containing the test mixture.

Reference sample: three glass reactors (3 liter capacity) each containing approximately 50 g of microcrystalline cellulose in addition to the test mixture.

Sample: three glass reactors (3 liter capacity) each containing approximately 50 g of sample in addition to the test mixture.

Test mixture: mature compost from an industrial composting plant mixed with an inert support (vermiculite, Sigma - Aldrich code 101532822), 300 g compost + 100 g vermiculite (dry weight) for each reactor.

Moisture of the test mixture: maintained at 50±5% throughout the test.

### Reactor ventilation system:

Through the use of aquarium pumps, ambient air is blown through silicone tubes into flow meters to regulate the flow which are placed before the reactor inlets. The air flows are maintained at values of approximately 10-15 liters/hour. The aerobic conditions allow the organic fraction of the sample to be converted into CO₂ during the test. The air is previously humidified by bubbling water set at the same temperature as the reactors.

At the reactor outlets, the air is conveyed through gas-impermeable pipes into a humidity recovery and elimination system. The dry air is then sent into an infrared gas analyzer which determines the concentration of CO₂ and the flow at regular time intervals. The percentage of biodegradability is calculated as the percentage of CO₂ produced compared to the theoretical total carbon content of the sample (total theoretical CO₂ production).

### Reactor incubation

Temperature during testing: 58±2°C.

CO₂ measurement: the CO₂ is measured twice a day for the first 5 days of testing, then at least once a day until the 45th day of testing. It is subsequently measured at least 5 days a week until the 90th day. The measurements are reduced to three times a week for tests which are extended for another 90 days.

Remixing and humidity restoration: for the entire duration of the test, the contents of the reactors are mixed once a week and water is also added if necessary (partially restoring the initial weight of the reactors). These operations are appropriately recorded.

**Table 1: Weight of the reactors at 0 and 99 days of testing.**

| Reactor | Initial weight (0 days) | | Final weight (99 days) | |
|---|---|---|---|---|
| | Gross | Net | Gross | Net |
| Blank_1 | 2151 | 785 | 2052 | 686 |
| Blank_2 | 2130 | 796 | 2035 | 701 |
| Blank_3 | 2139 | 777 | 2044 | 682 |
| Microcrystalline cellulose_1 | 2264 | 896 | 2132 | 764 |
| Microcrystalline cellulose_2 | 2241 | 889 | 2137 | 785 |
| Microcrystalline cellulose_3 | 2225 | 909 | 2079 | 763 |
| Compact feminine tampon applicators EarthBi AB313_LT_1 | 2253 | 893 | 2145 | 785 |
| Compact feminine tampon applicators EarthBi AB313_LT_2 | 2264 | 894 | 2164 | 794 |
| Compact feminine tampon applicators EarthBi AB313_LT_3 | 2270 | 900 | 2174 | 804 |

### Analytical methods

### Weighings

Three different scales were used during the test:
- Orma BCA 200S (max. 200 g, d=0.1 mg), for measuring volatile solids and humidity and for weighing the sample and the reference to be tested;
- Orma BC 1000 (max. 1000 g, d=0.01 g) for weighing samples with large volumes;
- Radwag PS 8100 (max. 8100 g, d=0.01 g) for weighing the inoculum, the vermiculite, the reactors, and the water added during the test.

### Dry weight and evaluation of humidity

The dry weight is determined in an oven at 105°C±2°C, 2 g of sample are treated for one night, then the sample is cooled in a desiccator and weighed in accordance with UNI 10780:1998 "Compost - Classification, requirements and use criteria".

### Evaluation of volatile solids

The volatile solids are determined on dry samples placed at 550°C for at least 4 hours until the complete disappearance of black particles in accordance with UNI ISO 1762:2015 "Paper, cardboard and pulp - Determination of the residue (ash) after incineration at 525°C", adopting a different temperature as required by EN 13432:20022/AC:2005.

### pH

The pH of the compost is measured using the HACH LANGE SensION+ PH3 pH meter, following calibration. The test is carried out in accordance with UNI EN ISO 14855-1:2013, paragraph 8, on a suspension of compost in deionized water with a ratio of 1:5 after mixing.

### Total nitrogen

The total nitrogen content in the compost is evaluated by means of the Kjeldahl method in accordance with UNI 10780:1998 "Compost - Classification, requirements and use criteria", applying the modifications indicated for the "Velp Scientifica UDK 149 Distiller" instruments.

1 g of sample is digested (mineralization: Velp Scientifica DK8 heating digester) in the presence of K₂SO₄, Se, H₂SO₄ concentrated for 30' at 420°C. After cooling, the digested fraction is distilled in the presence of NaOH and boric acid. The nitrogen trapped via boric acid is titrated as standard HCl. The results are expressed as %.

### Evaluation of CO₂ in the gas exiting the reactors

The concentration of CO₂ in the gas exiting the reactors is evaluated using a specific scanning system equipped with an NDIR detector for CO₂ analysis (Ecocontrol model EC100). The system measures and records the concentration of CO₂ and the flow exiting each reactor at pre-set time intervals. The CO₂ determination system is periodically calibrated using a standard gas mixture (CO₂/N) certified by an LAT calibration center; the system's flow meter is also calibrated by an LAT center.

### TOC (Total Organic Carbon) evaluation

The TOC is evaluated by an external laboratory in accordance with UNI EN 13137:2002 and the test is accredited by Accredia.

### RESULTS

### Validation of the biodegradability test

**Table 2: Validation of the test**

| | Average value | Yes | No |
|---|---|---|---|
| Was the degree of biodegradability of the reference material (microcrystalline cellulose) > 70% after 45 days? | 96.8 | X | |
| Is the difference between the percentage of biodegradability of the reference material (microcrystalline cellulose) in the different reactors < 20% at the end of the test? | 6.8 | X | |
| Is the CO₂ production of the compost after 10 days of testing between 50 and 150 mg CO₂/g of volatile solids? | 52.7 | X | |
| Has test validation been obtained? | | X | |

### Visual observations during the test

During the test setup, the ground sample was clearly visible compared to the rest of the mixture and much more voluminous than that of the reference reactors. After about 15 days the color of the sample began to darken and the volume began to reduce appreciably. After approximately 60 days the sample had disappeared and both the appearance and volume were similar to that of the reference sample.

### Total cumulative CO₂ production

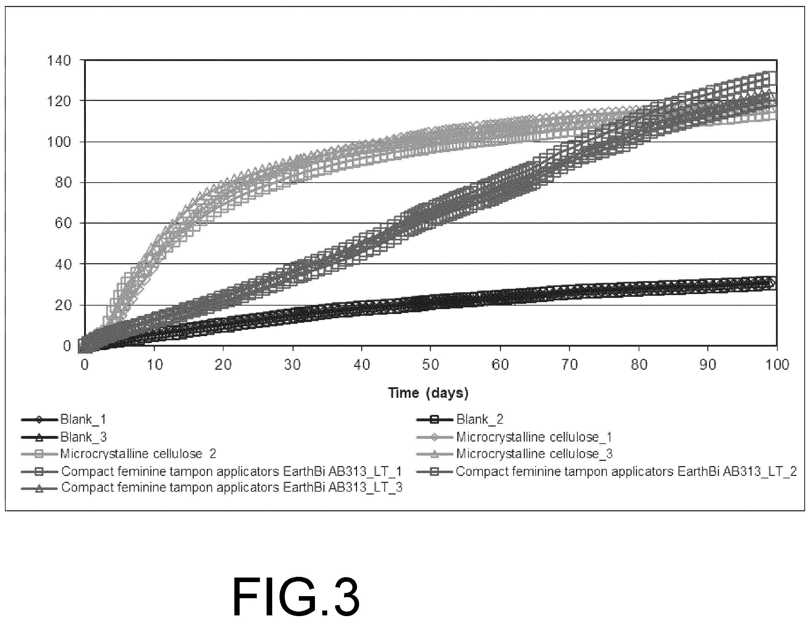

The total cumulative CO₂ production is shown in Figure 3 for the blank, reference and sample reactors. The CO₂ production of the "Compact feminine tampon applicators EarthBi AB313_LT" sample in the first few days was lower than the production of the reference microcrystalline cellulose. After 50 days, the CO₂ production of the "Compact feminine tampon applicators EarthBi AB313_LT" sample increased rapidly, reaching and exceeding that of the reference at the end of the test.

The following table shows the quantity of CO₂ produced after 99 days of testing:

| Reactor | Total CO₂ | Net CO₂ |
|---|---|---|
| Blank_1 | 29.05 | - |
| Blank_2 | 29.32 | - |
| Blank_3 | 29.62 | - |
| Microcrystalline cellulose_1 | 116.72 | 87.39 |
| Microcrystalline cellulose_2 | 111.47 | 82.14 |
| Microcrystalline cellulose_3 | 114.53 | 85.19 |
| Compact feminine tampon applicators EarthBi AB313_LT_1 | 131.17 | 100.28 |
| Compact feminine tampon applicators EarthBi AB313_LT_2 | 120.93 | 90.03 |
| Compact feminine tampon applicators EarthBi AB313_LT_3 | 123.09 | 92.20 |

Figure 3 shows the curves of CO₂ produced (in grams) by the syringe of the applicator of the invention, in comparison with the reference microcrystalline cellulose.

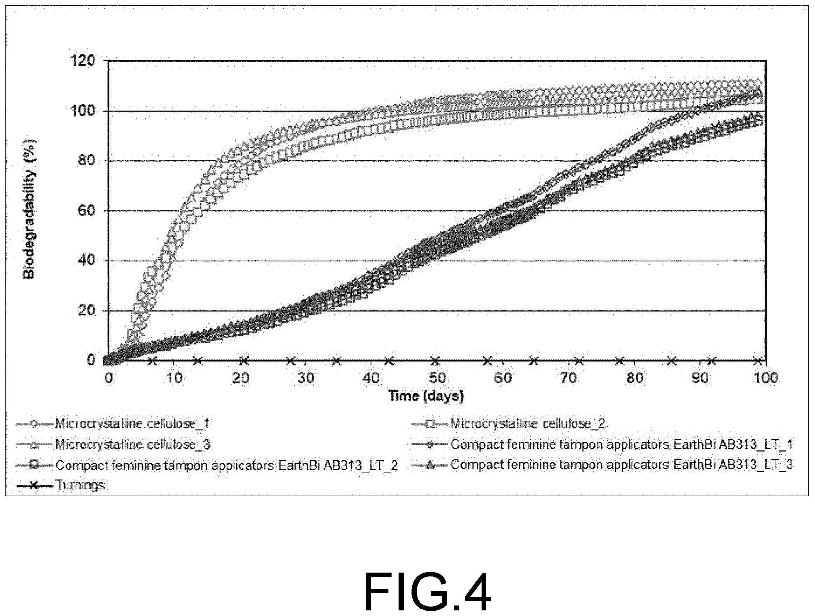

The biodegradation percentages of both the sample under analysis and the reference are determined by the ratio between the quantity of gaseous carbon produced (CO₂) compared to the initial organic carbon content of the sample input into the reactors. Figure 4 shows the trends in the biodegradability percentages obtained with the different replicates for the sample and the reference. The biodegradability rate of the "Compact feminine tampon applicators EarthBi AB313_LT" sample at the beginning of the test was very low compared to that of the reference microcrystalline cellulose. After the halfway mark of the test, the biodegradability rate of the sample increased quickly, reaching the reference. At the end of the test the "Compact feminine tampon applicators EarthBi AB313_LT" sample reached an average biodegradation value of 100.3±5.7%, therefore reaching a value ≥ 90% which represents the limit required by the EN 13432:20022 standard Annex A.2.2.2/AC:2005.

Figure 4 illustrates the aerobic biodegradability trend, in controlled compostability conditions, for the material making up the syringe 2 in comparison with the microcrystalline cellulose sample.

The following table shows the percentages of biodegradability at the end of the test (99 days), calculated with respect to the amount of TOC initially contained in the samples:

| Sample | Biodegradability (%) | | | | |
|---|---|---|---|---|---|
| | Replicate 1 | Replicate 2 | Replicate 3 | Average | Standard deviation |
| Reference Microcrystalline cellulose | 111.0 | 104.7 | 108.4 | 108.0 | 3.2 |
| Compact feminine tampon applicators EarthBi AB313_LT | 106.9 | 96.2 | 97.8 | 100.3 | 5.7 |

The test results show that, after the 99-day test, the material making up the syringe 2 of the applicator 1 of the invention reached an average biodegradation value of 100.3 ± 5.7%, compliant with the EN 13432:2002 standard Annex A.2.2.2/AC:2005 (required limit value greater than or equal to 90%).

For the production of the applicator of the invention, an injection molding process is used for the cylinder and plunger of the syringe with a material comprising at least polylactic acid, polybutyrate, polybutylene succinate and talc, preferably a material composed essentially of 3% to 10% by weight of polylactic acid, 34% to 60% by weight of polybutyrate, 10% to 15% by weight of polybutylene succinate, 20% to 30% by weight of talc and 7% to 11% by weight of epoxidized soybean oil and/or acetyl tributyl citrate and calcium stearate.

This material which makes up the syringe of the invention can be processed like any plastic and has the same structural and mechanical characteristics as a syringe of a conventional applicator, while eliminating any environmental pollution and maintaining the necessary structural resistance.

## Claims

1. An applicator for feminine hygiene tampons, said applicator (1) comprising a syringe (2) for expelling an absorbent tampon (3), said syringe having a cylinder (4) for containing said tampon and a plunger (5) for expelling said tampon from said cylinder, **characterized in that** said cylinder and/or said plunger are made of a biodegradable and compostable material which comprises 3% to 10% by weight of polylactic acid, 34% to 60% by weight of polybutyrate and 10% to 15% by weight of polybutylene succinate.

2. The applicator according to claim 1, **characterized in that** said biodegradable material further comprises 20% to 30% by weight of talc and 7% to 11% by weight of epoxidized soybean oil and/or acetyl tributyl citrate and calcium stearate.

## Patentansprüche

1. Ein Applikator für Tampons für die Frauenhygiene, wobei der genannte Applikator (1) eine Spritze (2) zum Ausstoßen eines saugfähigen Tampons (3) umfasst, wobei die genannte Spitze einen Zylinder (4) zur Aufnahme des genannten Tampons sowie einen Kolben (5) zum Ausstoßen des genannten Tampons aus dem genannten Zylinder hat, **dadurch gekennzeichnet, dass** der genannte Zylinder und/oder der genannte Kolben aus biologisch abbaubarem und kompostierbarem Material bestehen, welches von 3% bis 10% Gew. an Polymilchsäure, von 34% bis 60% Gew. an Polybutyrat und von 10% bis 15% Gew. an Polybutylensuccinat umfasst.

2. Der Applikator gemäß Anspruch 1, **dadurch gekennzeichnet, dass** das genannte biologisch abbaubare Material desweiteren von 20% bis 30% Gew. an Talkum und von 7% bis 11% Gew. an epoxidiertem Sojabohnenöl und/oder Acetyltributylzitrat und Kalziumstearat enthält.

## Revendications

1. Applicateur pour tampons d'hygiène féminine, ledit applicateur (1) comprenant une seringue (2) pour expulser un tampon absorbant (3), ladite seringue ayant un cylindre (4) pour contenir ledit tampon et un piston (5) pour expulser ledit tampon dudit cylindre, **caractérisé en ce que** ledit cylindre et/ou ledit piston sont composés d'une matière biodégradable et compostable qui comprend 3 % à 10 % en poids d'acide polylactique, 34 % à 60 % en poids de polybutyrate et 10 % à 15 % en poids de polybutylène succinate.

2. Applicateur selon la revendication 1, **caractérisé en ce que** ladite matière biodégradable comprend en outre 20 % à 30 % en poids de talc et 7 % à 11 % en poids d'huile de soja époxydée et/ou d'acétyl tributyle citrate et stéarate de calcium.
